# FASCICULE DE BREVET EUROPEEN

(11) **EP 1 313 416 B1**
(45) Date de publication et mention de la délivrance du brevet: **22.02.2006**
(21) Numéro de dépôt: 01969846.3
(22) Date de dépôt: 31.08.2001
(51) Int. Cl.: A61F 9/007

(54) **DRAIN A GLAUCOME**
DRAINAGERÖHRE ZUR BEHANDLUNG DES ERHÖHTEN INNENDRUDRUCKS
GLAUCOMA DRAIN

(30) Priorité: 01.09.2000 FR 0011190
(43) Date de publication de la demande: 28.05.2003
(73) Titulaire: Ioltechnologie-Production, 17180 Perigny (FR)
(72) Inventeur: DAHAN, Elie, Johannesburg (ZA)
(74) Mandataire: Santarelli
(86) Numéro de dépôt international: PCT/FR2001/002714
(87) Numéro de publication internationale: WO 2002/017832

(56) Documents cités:
- WO-A-95/08310
- WO-A-95/35078
- US-A- 4 521 210
- US-A- 4 787 885
- US-A- 5 601 094

## Description

La présente invention concerne un drain à glaucome pour faciliter l'écoulement de l'humeur aqueuse à travers la membrane trabéculo-descémétique.

Le glaucome est une maladie chronique, progressive et irréversible d'une pression intraoculaire trop élevée. Lorsqu'il existe un obstacle à l'évacuation de l'humeur aqueuse de la chambre antérieure à travers un filtre appelé trabéculum, la pression intraoculaire augmente et provoque la destruction progressive des fibres nerveuses. Il existe des traitements médicaux dont l'efficacité s'avère insuffisante dans de nombreux cas où, seule, la chirurgie peut permettre de réduire la pression intraoculaire.

L'intervention chirurgicale pour le traitement des glaucomes a pour but de créer un mécanisme de réduction de la pression intraoculaire. A ce jour, il y a deux principales catégories d'interventions de drainage, pénétrante et non pénétrante. La chirurgie la plus usuelle est la chirurgie pénétrante et s'appelle trabéculectomie. Elle consiste à créer une fistule entre la chambre antérieure de l'oeil et un espace sous-conjonctival. Cette intervention nécessite, outre l'ouverture de la chambre, par réalisation d'un trou dans la sclère à l'aide d'un instrument emporte-pièce, d'où des complications qui mènent à un nombre important d'échecs.

Parmi les catégories des interventions chirurgicales non pénétrantes figurent la viscocanalostomie et la sclérectomie profonde, également appelée trabéculectomie ab extemo. La viscocanalostomie du Dr Robert Stegmman (J Cataract Refract Surg, vol. 25, 1999) comprend la réalisation à un volet conjonctival à base fornix et la taille d'un premier volet parabolique d'un tiers de l'épaisseur sclérale, et d'un second volet parabolique scléral profond de presque deux tiers de l'épaisseur sclérale qui est excisé ultérieurement, l'ablation du plafond du canal de Schlemm et l'injection de sodium hyaluronate de haute viscosité non réticulé dans le canal de Schlemm et par la suite sous le volet extérieur après suture. Un réservoir scléral rempli de sodium hyaluronate est ainsi formé sous le volet extérieur. La porosité physiologique du trabéculum juxtacanaliculaire et la membrane descémétique permet l'évacuation de l'humeur aqueuse et la réduction de la pression intraoculaire. Mais le sodium hyaluronate s'élimine dans un délai de cinq à six jours et le réservoir est rapidement comblé par la fibrose limitant la durée de l'efficacité de l'intervention.

Un développement de cette technique est présenté dans la demande PCT WO 98/35640 qui décrit un implant de scléro-kératectomie pré-descémétique réalisé en acide hyaluronique réticulé présentant la forme d'un prisme à base triangulaire. Cet implant est censé occuper plus durablement l'espace créé chirurgicalement mais le volume de l'implant se réduit de moitié en quatre mois et est totalement résorbé par la suite.

Selon une autre variante de la sclérectomie profonde du Dr Mermoud, un drain à glaucome de forme cylindrique est réalisé à partir de collagène de porc lyophilisé et suturé dans le lit scléral profond. Le drain transporte l'humeur aqueuse par capillarité. Le volet scléral superficiel ne referme pas l'extrémité postérieure du drain cylindrique qui est inadapté à la configuration du réservoir scléral. Le drain est résorbé dans les mois qui suivent l'intervention.

L'ensemble de ces implants et drains à glaucome réalisés en acide hyaluronique réticulé et non réticulé ou en collagène sont tous résorbables et donc ne constituent pas une solution réellement durable de drainage de l'humeur aqueuse de la chambre antérieure et partant, une diminution de la pression intraoculaire assurée dans le temps. En outre, ces implants et drains d'origine animale ont un coût de fabrication élevé et comporte un risque de transmission de maladies, notamment virales, et lorsqu'ils sont d'origine porcine ou traités à l'héparine porcine, les chirurgiens se heurtent à des refus des patients pour des motifs religieux.

La demande WO 95/35078 décrit un implant de sclérotomie avec ou sans incision du trabéculum réalisé en copolymère méthylméthacrylate vynylpyrrolidone qui présente un taux d'hydrophilie élevé de l'ordre de 40 %. Il comporte une partie intra-sclérale adaptée à être disposée contre le

L'extrémité externe de l'implant déverse l'humeur aqueuse sous la conjonctive et forme une bulle visible à travers la conjonctive.

Le document US-4.521.210 décrit un drain à glaucome pour sclérectomie pénétrante réalisé en matériau semi-rigide biocompatible, tel que le polyméthylmethacrylate. Le drain est de préférence cruciforme. Son extrémité avant pénètre dans la chambre antérieure par l'angle irido-cornéen. Sa partie centrale s'étend entre la sclérotique et le corps ciliaire et à travers une partie de la sclérotique à laquelle il est suturé. L'extrémité arrière du drain pénètre à l'intérieur d'un espace suprachoroidal où les fluides ainsi drainés de la chambre antérieure sont absorbés.

L'objet de l'invention est un drain à glaucome qui est susceptible d'assurer le drainage pour des durées prolongées, voire indéfiniment et dépourvus des inconvénients des drains à glaucome et autres implants de sclérotomie connus.

Selon un premier aspect de l'invention, un drain à glaucome pour la sclérectomie profonde non pénétrante réalisé en matière synthétique non résorbable et hydrophile, le drain étant conformé pour être entièrement recouvert par le volet scléral et complètement inséré dans l'espace intrascléral, le drain comportant une barre transversale à une extrémité avant du drain et un tronc longitudinal s'étendant longitudinalement à partir de ladite barre transversale, et les extrémités opposées de la barre transversale étant configurées pour pénétrer à l'intérieur du canal de Schlemm.

Grâce à l'hydrophilie du drain, il participe lui même au passage de l'humeur aqueuse depuis la membrane trabéculo-descémétique dans l'espace Intrascléral où elle est reprise par la circulation veineuse. Logé complètement dans l'espace intra-scléral, il ne promeut pas la formation de bulles sous la conjonctive.

De préférence la matière non résorbable et hydrophile et également apyrogène et, bien entendu, biocompatible, est en polyhydroxyéthyl methacrylate 38% (PolyHEMA 38%) ou un acrylique hydrophile, Outre le fait que ces matières ne sont pas résorbables, Ils ne provoquent pas de fibrose tels que d'autres matériaux biocompatlbles et non résorbable, et notamment le silicone. Par conséquent, le drain permet de garder autour de lui un espace intrascléral permanent et sous le volet scléral propice à l'écoulement de l'humeur aqueuse à durée prolongée. Le site de drainage alors peu fibrosé peut être révisé, notamment pour rétablir l'écoulement de l'humeur aqueuse.

De préférence, le présent drain à glaucome comporte une barre transversale et un tronc qui s'étend longitudinalement à partir de la barre transversale et est monobloc.

Selon une forme de réalisation préférée, le drain a une forme en T comprenant la barre transversale disposée à l'extrémité avant et le tronc longitudinal qui s'étend vers l'arrière à partir du milieu de la barre transversale.

Selon une autre caractéristique préférée, la barre transversale comporte sur la surface postérieure une paire de saillies de part et d'autre d'une zone médiane qui forme une cuvette d'écoulement.

Pour une sclérectomie profonde non pénétrante, la barre transversale longe et recouvre en partie la membrane trabéculo-descémétique et les extrémités de la barre transversale passent sous la sclère intacte et à l'intérieur du canal de Schlemm. Dans cette position, le bord avant du drain est à proximité de, et en pratique en contact avec la membrane trabéculo-descémétique. La longueur du tronc est telle que son extrémité arrière ne s'étend pas au-delà de l'espace intra-scléral.

Selon une deuxième forme de réalisation, au moins une partie du bord avant du drain est bombé, de sorte que, lorsque les bras de la barre transversale sont au droit de la membrane trabéculo-descémétique, le bord avant bombé applique une tension sur sur cette membrane afin d'en étirer les mailles et d'augmenter sa porosité, et partant, l'écoulement de la chambre antérieure vers l'espace intra-scléral.

De préférence, un trou est aménagé entre les bras de la barre transversale et constitue une cible pour placer des micro-ponctions par laser YAG dans la membrane trabéculo-descémétique lorsqu'elles s'avèrent nécessaires pour améliorer l'évacuation de l'humeur aqueuse vers l'espace intra-scléral.

Les caractéristiques et les avantages de l'invention ressortiront d'ailleurs de la description qui va suivre à titre d'exemple en référence aux dessins annexés sur lesquels :
- la figure 1 est une vue en perspective d'un drain à glaucome selon une première forme de réalisation de l'invention ;
- la figure 2 est une vue du drain illustré sur la figure 1 logé dans l'espace intra-scléral implanté avant la fermeture du volet scléral superficiel ;
- la figure 3 est un détail de la figure 2 ;
- la figure 4 est une vue en perspective d'un drain à glaucome selon une deuxième forme de réalisation de l'invention ;
- la figure 5 est une vue du drain illustré sur la figure 4 logé dans l'espace intra-scléral ;
- la figure 6 est une vue en perspective d'un drain à glaucome selon une troisième forme de réalisation.

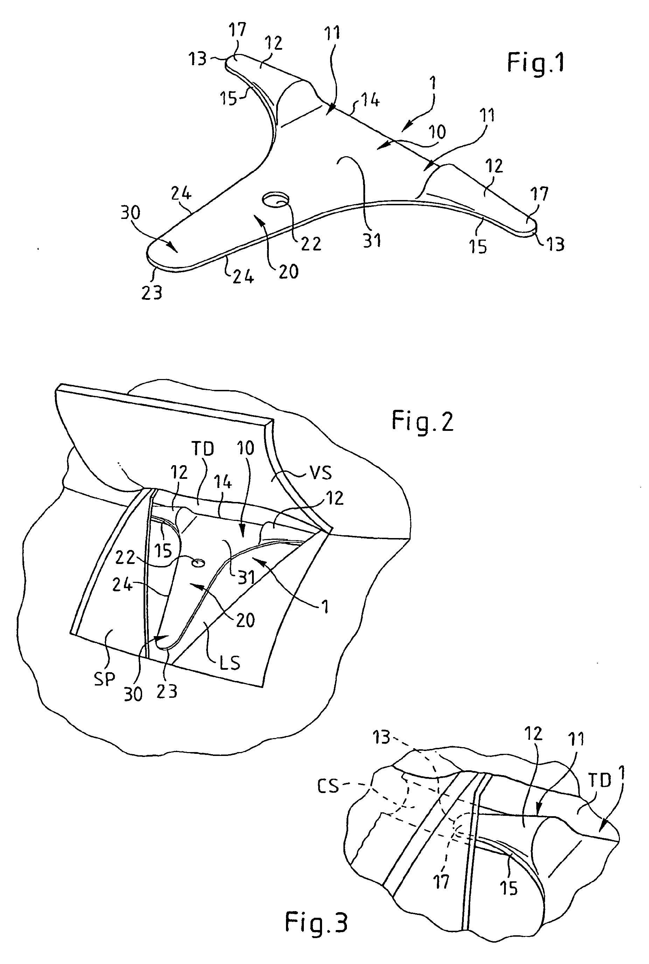

Le drain à glaucome 1, tel qu'illustré à la figure 1, est destiné à être implanté dans le lit scléral profond (LS) dans le cadre d'une sclérectomie profonde non pénétrante (voir figures 2 et 3).

Selon la présente invention, le drain à glaucome est réalisé en une matière biocompatible, non résorbable, hydrophile et apyrogène. Cette matière hydrophile est perméable à l'humeur aqueuse, de sorte que l'humeur aqueuse puisse transiter par le drain, notamment de son extrémité, dite avant, à proximité de, et en pratique en contact avec la membrane trabéculo-descémétique vers son extrémité, dite arrière pour récupération par le système veineux. Le taux d'hydrophilie est de préférence élevé. Le polyhydroxy éthyl méthacrylate (PolyHEMA) 38% et le copolymère acrylique hydrophile, tel que celui commércialisé par la société loltech sous la marque POLY-MEGMA™, donnent satisfaction.

Un tel implant permet un drainage in situ pendant une durée prolongée, de plusieurs années, voire indéfiniment, et ce sans modification de la géométrie du drain puisque la matière constitutive est non résorbable dans l'environnement d'implantation. Le matériau hydrophile dans lequel le drain est de préférence réalisé ne provoque pas la fibrose et de ce fait permet de garder autour de lui un espace intra-scléral propice à l'écoulement de l'humeur aqueuse. De même, le site étant peu fibrosé, il peut être révisé en cas de besoin.

Le drain 1 comprend une barre transversale 10 et un tronc longitudinal 20. La barre transversale 10 est située à une extrémité dite avant et comprend une paire de bras 11 s'étendant dans les sens opposés. Le tronc longitudinal 20 s'étend longitudinalement vers l'arrière à partir de la barre transversale. L'axe général de la barre transversale 10 et celui du tronc longitudinal 20 sont donc sensiblement perpendiculaires l'un à l'autre. Le tronc longitudinal s'étend de préférence à partir du milieu de cette dernière et de ce fait, le drain 1 a la forme générale d'un T.

Le contour du drain est défini par des bords de la barre transversale 10 et du tronc longitudinal 20 et comporte un bord avant 14 de la barre transversale 10 s'étendant entre les bouts arrondis 13 de la barre qui sont prolongés par les bords latéraux 15 de l'autre côté de chacun des bras et ensuite par les bords latéraux 24 du tronc longitudinal 20 qui se terminent par un bout arrondi 23 à l'extrémité arrière du tronc longitudinal 20.

La face antérieure, non illustrée, est sensiblement plane et donc sans relief. Il en est de même pour la surface postérieure 30 du tronc longitudinal 20 et de la zone médiane 31 de la barre transversale 10 entre les bras 11.

Selon une forme de réalisation préférée, la face postérieure des bras 11 comporte une saillie 12, de forme générale effilée et de préférence semi-tronconique dont le sommet se trouve au bout arrondi 13 des bras respectifs.

La zone médiane 31 disposée entre les saillies 12 forme entre elles une cuvette apte à diriger l'écoulement sur la surface postérieure le long du tronc longitudinal 20 vers l'arrière.

Une ouverture 22, de préférence circulaire, est pratiquée à mi-hauteur du tronc 20 et sur l'axe longitudinal de celui-ci.

En pratique, la largeur de la barre transversale 10 est légèrement plus grande que la largeur du volet scléral profond enlevé, de sorte que les parties terminales 17 des bras 11 puissent s'introduire sous la sclère intacte à l'intérieur du canal de Schlemm (CS), tel qu'illustré à la figure 2 et dans le détail de la figure 3. La prise de la partie terminale 17 des bras respectifs assure un bon positionnement et orientation de la barre transversale 10 par rapport au canal de Schlemm (CS) et la membrane trabéculo-descémétique. On remarque par ailleurs que la barre transversale 10 longe et recouvre la membrane trabéculo-descémétique, l'ablation du plafond du canal de Schlemm ayant été effectuée précédemment, tel que positionné par rapport au canal de Schlemm, et le bord avant de la barre transversale est en contact sur toute sa longueur avec la membrane trabéculo-descémétique (voir figure 2).

Une fois les parties terminales 17 des bras respectifs en place, le tronc 20 peut être suturé au lit scléral (LS) à la faveur de l'ouverture 22. Le volet scléral superficiel (VS) peut alors être rabattu et suturé et ensuite la membrane conjonctivale.

Le drain selon l'invention assure une évacuation de l'humeur aqueuse dans l'espace intra-scléral formé sous le volet scléral, d'une durée prolongée grâce à son caractère non résorbable d'une part, et hydrophile d'autre part. Le drain permet de garder autour de lui une zone libre permanente d'écoulement. Du fait de l'hydrophilie élevée du polyHEMA 38 % ou de l'acrylique hydrophile, l'humeur aqueuse peut aussi transiter par le drain vers le système veineux. L'écoulement de l'humeur aqueuse est dirigé de l'extrémité avant vers l'extrémité arrière est favorisé grâce à la cuvette définie entre les saillies sur la face postérieure de la barre transversale. La configuration et la taille du drain permettent une implantation entièrement sous le volet scléral et complètement dans l'espace intra-scléral.

En cas de besoin, l'écoulement de l'humeur aqueuse peut être augmentée en pratiquant des micro-ponctions par laser YAG aux environs du corps du drain 2 au niveau du canal de Schlemm (CS). D'ailleurs, la réalisation d'une fenêtre dans le canal de Schlemm et le positionnement du drain qui en résulte, facilitent la localisation des micro-ponctions.

Toutefois, selon une forme de réalisation non illustrée, les saillies peuvent être éliminées de sorte que la face postérieure du drain soit sensiblement plane et donc sans relief, comme la face antérieure. De même, bien que la forme en T, telle qu'illustrée, soit préférée, d'autres formes du drain peuvent être adoptées. En tout cas, le bord avant doit être positionné tel qu'illustré sur la figure 2, c'est-à-dire à proximité de et en pratique en contact avec la membrane trabéculo-descémétique.

Dans l'état hydraté du drain, la largeur de la barre transversale 10 entre ses extrémités 13 opposées est de préférence 3 à 4 mm, voire plus, et plus particulièrement environ 4 mm. La hauteur de la barre transversale définie entre le bord avant et le bord latéral opposé est de l'ordre de 0,20 à 0,50 mm et de préférence environ 0,25 mm. La hauteur totale du drain se situe de préférence entre 2,50 et 3,50 mm et en tout cas inférieure à 4 mm, et la hauteur du tronc est d'environ 2,75 mm.

Le drain est de préférence mince avec une épaisseur de l'ordre de 0.1 mm en dehors de celle de la saillie qui est de l'ordre de 0,30 mm. Grâce à cette faible épaisseur et à la matière constitutive, le drain peut s'adapter à la courbure de la surface du lit scléral. L'épaisseur du drain étant de l'ordre de celle du volet scléral profond excisé, le drain peut se loger entièrement dans l'espace intra-scléral après fermeture du volet scléral superficiel.

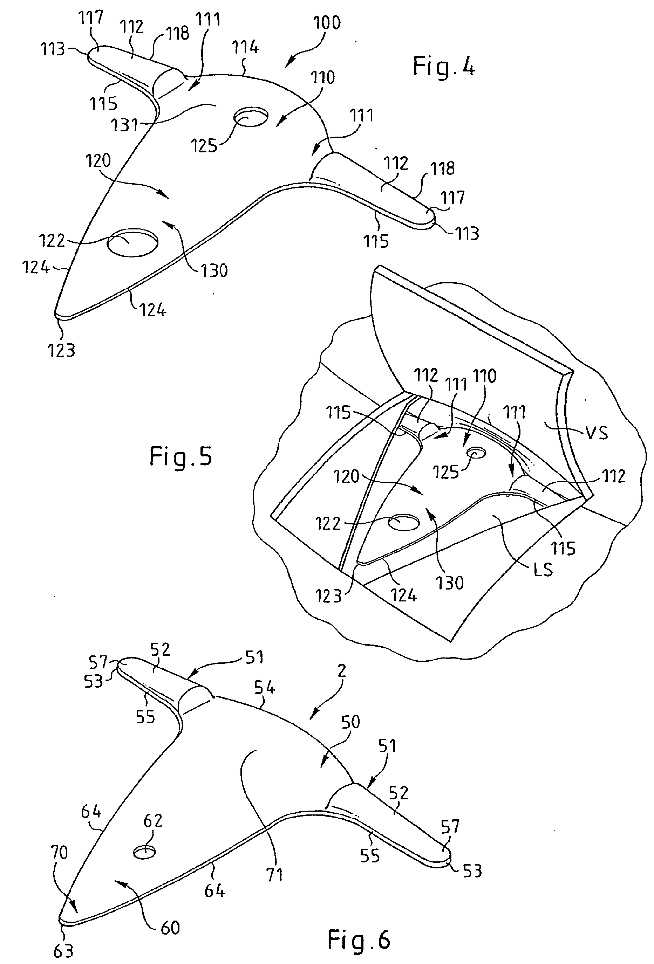

Le drain à glaucome 100 selon une deuxième forme de réalisation, tel qu'illustré à la figure 4, est également destiné à être implanté dans le lit scléral profond (LS) dans le cadre d'une sclérectomie profonde non pénétrante (voir figure 5).

Le drain à glaucome 100 est également réalisé en la même matière biocompatible, non résorbable, hydrophile et apyrogène que celui selon la première forme de réalisation et permet la même implantation, avec les mêmes fonctions et avantages que ceux de la première forme de réalisation.

Le drain 100 comprend une barre transversale 110 et un tronc longitudinal 120. La barre transversale 110 est située à une extrémité dite avant et comprend une paire de bras 111 s'étendant dans les sens opposés. Le tronc longitudinal 120 s'étend longitudinalement à une partie de la barre transversale. L'axe général de la barre transversale 110 et celui du tronc longitudinal 120 sont donc sensiblement perpendiculaires l'un à l'autre. Le tronc longitudinal s'étend de préférence à partir du milieu de cette dernière et de ce fait, le drain 100 a la forme générale d'un T.

Le contour du drain est défini par des bords de la barre transversale 110 et du tronc longitudinal 120 et comporte un bord avant bombé 114 de la barre transversale 110 entre les bras prolongé jusqu'aux bouts arrondis 113 de la barre par des bords avant latéraux rectilignes 118 des bras. Les bouts arrondis 113 sont prolongés par les bords latéraux 115 parallèles aux bords avant latéraux 118, de l'autre côté de chacun des bras et ensuite par les bords latéraux 124 du tronc longitudinal 120 qui se terminent par un bout légèrement arrondi 123 à l'extrémité arrière du tronc longitudinal 120.

La face antérieure, non illustrée, est sensiblement plane et donc sans relief. Il en est de même pour la surface postérieure 130 du tronc longitudinal 120 et de la zone médiane 131 de la barre transversale 110.

La face postérieure du drain comporte de part et d'autre de la zone médiane 131, une saillie 112, de forme générale effilée et de préférence semi-tronconique dont le sommet se trouve au bout arrondi 113 des bras respectifs.

La zone médiane 131 disposée entre les saillies 112 forme entre elles une cuvette apte à diriger l'écoulement sur la surface postérieure entre les saillies 112 et le long du tronc longitudinal 120.

Une ouverture 122, de préférence circulaire, est pratiquée dans le tronc 120 à environ trois quarts de la distance entre le centre du bord antérieur et l'extrémité postérieure du tronc et sur l'axe longitudinal de celui-ci.

En pratique, la largeur de la barre transversale 110 est légèrement plus grande que la largeur du volet scléral profond enlevé, de sorte que les parties terminales 117 des bras 111 puissent s'introduire sous la sclère intacte à l'intérieur du canal de Schlemm (CS), tel qu'illustré à la figure 5. La prise de la partie terminale 117 des bras respectifs assure un bon positionnement et orientation de la barre transversale 110 par rapport au canal de Schlemm (CS) et la membrane trabéculo-descémétique. La barre transversale 110 longe et recouvre en partie la membrane trabéculo-descémétique, l'ablation du plafond du canal de Schlemm ayant été effectuée précédemment. Tel que positionné par rapport au canal de Schlemm, au moins le bord avant bombé 114 de la barre transversale est en contact avec la membrane trabéculo-descémétique, (voir figure 5) et le bord avant bombé 114 la met sous tension, de sorte que ses mailles en s'étirant augmentent l'écoulement de l'humeur aqueuse à travers la membrane trabéculo-descémétique.

Une fois les parties terminales 117 des bras respectifs en place, le tronc 120 peut être suturé au lit scléral (LS) à la faveur de l'ouverture 122. Le volet scléral superficiel (VS) peut alors être rabattu et suturé et ensuite la membrane conjonctivale.

Le drain selon cette deuxième forme de réalisation assure l'évacuation de l'humeur aqueuse dans l'espace intra-scléral et améliore son écoulement à travers la partie de la membrane trabéculo-descémétique mise sous tension.

En cas de besoin, l'écoulement de l'humeur aqueuse peut être augmentée en pratiquant des micro-ponctions par laser YAG. A cette fin, un second trou 125 est aménagé entre les bras 112 et sensiblement aligné avec ceux-ci. Ce trou constitue un point de repère et une cible au travers de laquelle le praticien pourra diriger le laser YAG afin de localiser les micro-ponctions au niveau de la membrane trabéculo-descémétique.

De préférence, la largeur de la barre transversale 110 entre ses extrémités 113 opposées est de 3,5 à 4 mm, voire plus, et de préférence environ 4 mm. La largeur de la zone médiane 113 entre les extrémités internes des saillies 112 est plus large dans cette forme de réalisation et en pratique de l'ordre de 1,7 mm. La hauteur de la barre transversale définie entre les bords latéraux parallèles 115, 118 est de l'ordre de 0,20 à 0,30 mm et de préférence environ 0,25 mm. La hauteur totale du drain se situe de préférence entre 3 et 3,75 mm, et de préférence, est d'environ 3,50 mm.

Le drain est de préférence mince avec une épaisseur de l'ordre de 0.15 mm en dehors de celle de la saillie qui est de l'ordre de 0,30 mm et suffisamment souple pour suivre la courbure de lit scléral (LS).

Dans une troisième forme de réalisation illustrée sur la figure 6, le drain à glaucome 2 comprend une barre transversale 50 et un tronc longitudinal 60. La barre transversale 50 est située à une extrémité dite avant et comprend une paire de bras 51 s'étendant dans les sens opposés. Le tronc longitudinal 60 s'étend longitudinalement à une partie de la barre transversale. L'axe général de la barre transversale 50 et celui du tronc longitudinal 60 sont donc sensiblement perpendiculaires l'un à l'autre. Le tronc longitudinal s'étend de préférence à partir du milieu de cette dernière et de ce fait, le drain 2 a également la forme générale d'un T.

Le contour du drain est défini par les bords de la barre transversale 50 et du tronc longitudinal 60 et comporte un bord avant 54 comprenant une partie convexe centrale de la barre transversale 50 s'étendant ensuite de manière rectiligne jusqu'aux bouts arrondis 53 de la barre. Le contour suit alors les bords latéraux 55 de l'autre côté de chacun des bras et ensuite par les bords latéraux 64 du tronc longitudinal 60 qui converge vers un petit bout arrondi 63 à l'extrémité arrière du tronc longitudinal 60.

Selon une troisième forme de réalisation, le drain comporte la face antérieure, non illustrée à la figure 6, est sensiblement plane et donc sans relief. Il en est de même pour la surface postérieure du tronc longitudinal 60 et de la zone médiane 71 de la barre transversale 50.

Selon une forme de réalisation préférée, la face postérieure 30 du drain 2 comporte de part et d'autre de la zone médiane 71, une saillie 52, de préférence de forme générale semi-tronconique dont le sommet se trouve au bout arrondi 53 des bras respectifs.

La zone médiane 71 disposée entre les saillies 52 se trouve alors dans le même plan que la surface postérieure 70 du tronc longitudinal 60.

Une ouverture 62, de préférence circulaire, est pratiquée aux deux tiers environ de la distance entre le bord 52 et le bout arrondi 63 et se trouve sur l'axe longitudinal du tronc.

De même, du fait de l'hydrophilie élevée du poly-HEMA 38 % ou l'acrylique hydrophile, l'humeur aqueuse peut également transiter par le drain lui-même vers le système veineux uvéoscléral. Enfin, comme dans les deux premières formes de réalisation, grâce au fait que le drain soit non résorbable et hydrophile, un espace permanent est constitué autour du tronc du drain, nonobstant la cicatrisation inévitable entre le lit scléral et le volet scléral superficiel.

Dans l'état hydraté, la largeur de la barre transversale 50 entre ses extrémités 53 opposées est de préférence 3,5 à 4,5 mm et plus particulièrement, de l'ordre de 3,5 mm. La longueur de chacun des bras de la barre transversale est de l'ordre de 1 mm. La hauteur de la barre transversale définie entre les bords opposés est de l'ordre de 0,2 à 0,4 mm et de préférence environ 0,25 mm. De même, la hauteur totale du drain se situe de préférence entre 3 et 4 mm, et plus particulièrement 3,5 mm. La largeur du drain au niveau de la fente pratiquée dans le canal de Schlemm est de préférence entre 1,3 et 1,8 mm et plus particulièrement de l'ordre de 1,5 mm, c'est-à-dire de l'ordre de 0,1 mm, plus petite que la largeur de la fente.

L'épaisseur du drain est de l'ordre de 0,1 mm, sauf au niveau des saillies où elle est de l'ordre de 0,30 mm.

Le drain à glaucome de cette troisième forme de réalisation peut donc être implanté de la même manière que selon la première forme de réalisation.

Quelle que soit la forme du drain à glaucome réalisé par exemple en polyHEMA 38 % ou un acrylique hydrophile, il peut être fabriqué selon des procédés usuels dans le domaine des implants intraoculaires. De préférence, le drain est usiné au tour ("lathe cut").

Une fois réalisé et stérilisé, ce drain est conditionné dans une poche ou fiole contenant une solution isotonique telle que le NaCl 0,9 %. Le drain peut être logé dans un support (non illustré) pour faciliter sa préhension.

Bien entendu, la présente invention ne se limite pas à la forme de réalisation décrite et représentée, mais englobe toute variante d'exécution. Bien que la forme en T de chacune des formes de réalisation illustrée soit préférée pour les deux applications décrites, d'autres configurations peuvent être adoptées. De même, d'autres matériaux non résorbables et hydrophiles peuvent être utilisés pour la réalisation d'implants intraoculaires souples peuvent être employés.

## Revendications

1. Drain à glaucome pour la sclérectomie profonde non pénétrante réalisé en matière synthétique non résorbable et hydrophile, le drain étant conformé pour être entièrement recouvert par le volet scléral et complètement inséré dans l'espace intra-scléral, le drain comportant une barre transversale (10, 50, 110) à une extrémité avant du drain et un tronc longitudinal (20, 60, 120) s'étendant longitudinalement à partir de ladite barre transversale, et les extrémités opposées de la barre transversale (10, 50_{⊥} 110) étant configurées pour pénétrer à l'intérieur du canal de Schlemm (CS).

2. Drain à glaucome selon la revendication 1, **caractérisé en ce que** la forme générale est celle d'un T.

3. Drain à glaucome selon l'une quelconque des revendications 1 ou 2, **caractérisé en ce que** le drain est réalisé en polyHEMA 38 % ou en acrylique hydrophile.

4. Drain à glaucome selon l'une quelconque des revendications précédentes, **caractérisé en ce que** la barre transversale (10, 50, 110) comporte au moins une saillie (12, 52, 112) sur la face postérieure (31, 71, 131) du drain.

5. Drain à glaucome selon la revendication 4, **caractérisé en ce qu'**une paire de saillies (12, 52, 112) est disposée sur les bras respectifs constitués par la barre transversale (10, 50, 110) et forment entre elles une cuvette d'écoulement.

6. Drain à glaucome selon la revendication 4 ou 5, **caractérisé en ce que** chacune des saillies (12, 52, 112) a une section qui s'effile vers le sommet situé près des parties terminales respectives de la barre transversale (10, 50).

7. Drain à glaucome selon l'une quelconque des revendications précédentes, **caractérisé en ce que** le tronc longitudinal (20, 60, 120) est effilé à partir de la barre transversale vers son extrémité libre.

8. Drain à glaucome selon l'une quelconque des revendications 2 à 7, **caractérisé en ce que** la barre transversale (10, 110) est configurée pour longer et recouvrir le canal de Schlemm (CS) et ses extrémités sont configurées également pour pénétrer sous la sclére profonde.

9. Drain à glaucome selon l'une quelconque des revendications précédentes, **caractérisé en ce que** la barre transversale (10, 50, 110) a une largeur comprise entre environ 3 et 4 mm.

10. Drain à glaucome selon l'une quelconque des revendications précédentes, **caractérisé en ce que** le drain comporte une ouverture (22, 72, 122) pour suturer le drain au volet scléral profond.

11. Drain à glaucome selon l'une quelconque des revendications précédentes, **caractérisé en ce que** la longueur totale du drain est comprise entre environ 2,5 et 3,50 mm.

12. Drain à glaucome selon l'une quelconque des revendications précédentes, **caractérisé en ce que** le bord avant (14) est sensiblement rectiligne et configuré pour être en contact sur toute sa largeur avec la membrane trabéculo-descémétique.

13. Drain à glaucome selon l'une quelconque des revendications 1 à 12, **caractérisé en ce que** le bord avant (54, 114) est saillant pour mettre la membrane trabéculo-descémétique sous tension.

14. Drain à glaucome selon l'une quelconque des revendications précédentes, **caractérisé en ce que** le bord avant a une partie convexe (54, 114).

15. Drain à glaucome selon la revendication 13, **caractérisé en ce que** le bord avant saillant (54, 114) est bombé et aménagé entre les bords avant des bras latéraux.

16. Drain à glaucome selon l'une quelconque des revendications précédentes, **caractérisé en ce qu'**un trou (125) est aménagé entre des bras de la barre (50) et constitue une cible pour placer des microponctions de laser.

## Claims

1. A glaucoma drain for deep non-penetrating scterectomy, made from hydrophilic non-resorbable synthetic material, the drain being configured to be entirely covered by the scleral flap and completely inserted inside the intrascleral space, the drain comprising a transverse bar (10, 50, 110) at a front end of the drain and a longitudinal body (20, 60, 120) extending longitudinally from said transverse bar, and the opposite ends of the transverse bar (10, 50, 110) being configured to penetrate inside Schlemm's canal (CS).

2. A glaucoma drain according to claim 1, **characterized in that** its general form is that of a T.

3. A glaucoma drain according to any one of claims 1 or 2, **characterized in that** the drain is produced from 38% polyHEMA or hydrophilic acrylic.

4. A glaucoma drain according to any one of the preceding claims, **characterized in that** the transverse bar (10, 50, 110) comprises at least one projection (12, 52, 112) on the posterior face (31, 71, 131) of the drain.

5. A glaucoma drain according to claim 4, **characterized in that** a pair of projections (12, 52, 112) is disposed on the respective arms constituted by the transverse bar (10, 50, 110) and forms a channel between them.

6. A glaucoma drain according to claim 4 or 5, **characterized in that** the section of each of the projections (12, 52, 112) tapers towards the apex situated near the respective terminal portions of the transverse bar (10, 50).

7. A glaucoma drain according to any one of the preceding claims, **characterized in that** the longitudinal body (20, 60, 120) tapers from the transverse bar towards its free end.

8. A glaucoma drain according to any one of the claims 2 to 7, **characterized in that** the transverse bar (10, 110) is configured to extend along and cover Schlemm's canal (CS) and its ends are also configured to penetrate under the deep sclera.

9. A glaucoma drain according to any one of the preceding claims, **characterized in that** the width of the transverse bar (10, 50, 110) is between approximately 3 and 4 mm.

10. A glaucoma drain according to any one of the preceding claims, **characterized in that** the drain comprises an opening (22, 72, 122) for suturing the drain to the deep scleral flap.

11. A glaucoma drain according to any one of the preceding claims, **characterized in that** the total length of the drain is between approximately 2.5 and 3.50 mm.

12. A glaucoma drain according to any one of the preceding claims, **characterized in that** the front edge (14) is substantially straight and is configured to be in contact over its entire length with the trabeculo-descemetic membrane.

13. A glaucoma drain according to any one of claims 1 to 12, **characterized in that** the front edge (54, 114) projects in order to tension the Descemet's membrane.

14. A glaucoma drain according to any one of the preceding claims, **characterized in that** the front edge has a convex portion (54, 114).

15. A glaucoma drain according to claim 13, **characterized in that** the projecting front edge (54, 114) is convex and is provided between the front edges of the lateral arms.

16. A glaucoma drain according to any one of the preceding claims, **characterized in that** a hole (125) is provided between arms of the bar (50) and constitutes a target for locating laser micro-punctures.

## Patentansprüche

1. Glaukom-Drainage für die nicht penetrierende tiefe Sklerektomie, die aus einem nicht resorbierbaren und hydrophilen Kunststoff hergestellt ist, wobei die Drainage so beschaffen ist, dass sie durch die Skleraklappe vollständig bedeckt und in den intraskleralen Raum vollständig eingeschoben ist, wobei die Drainage ein transversales Glied (10, 50, 110) an einem vorderen Ende der Drainage und einen longitudinalen Schaft (20, 60, 120), der sich longitudinal von dem transversalen Glied erstreckt, umfasst, wobei die gegenüberliegenden Enden des transversalen Gliedes (10, 50, 110) so konfiguriert sind, dass sie in den Schlemmschen Kanal (CS) eindringen.

2. Glaukom-Drainage nach Anspruch 1, **dadurch gekennzeichnet, dass** die allgemeine Form jene eines T ist.

3. Glaukom-Drainage nach einem der Ansprüche 1 oder 2, **dadurch gekennzeichnet, dass** die Drainage aus polyHEMA 38 % oder aus hydrophilem Acryl verwirklicht ist.

4. Glaukom-Drainage nach einem der vorhergehenden Ansprüche, **dadurch gekennzeichnet, dass** das transversale Glied (10, 50, 110) auf der hinteren Fläche (31, 71, 131) der Drainage wenigstens einen Vorsprung (12, 52, 112) aufweist.

5. Glaukom-Drainage nach Anspruch 4, **dadurch gekennzeichnet, dass** auf den entsprechenden Armen, die durch das transversale Glied (10, 50, 110) gebildet sind, ein Paar Vorsprünge (12, 52, 112) angeordnet ist, die miteinander eine Ablaufwanne bilden.

6. Glaukom-Drainage nach Anspruch 4 oder 5, **dadurch gekennzeichnet, dass** jeder der Vorsprünge (12, 52, 112) einen Querschnitt besitzt, der zum Scheitel, der sich in der Nähe der jeweiligen Anschlussteile des transversalen Gliedes (10, 50) befindet, konisch zuläuft.

7. Glaukom-Drainage nach einem der vorhergehenden Ansprüche, **dadurch gekennzeichnet, dass** der longitudinale Schaft (20, 60, 120) ausgehend von dem transversalen Glied zu seinem freien Ende konisch zuläuft.

8. Glaukom-Drainage nach einem der Ansprüche 2 bis 7, **dadurch gekennzeichnet, dass** das transversale Glied (10, 110) so konfiguriert ist, dass es den Schlemmschen Kanal (CS) entlanggeführt ist und diesen abdeckt und dass seine Enden außerdem so konfiguriert sind, dass sie unter die tiefe Sklera eindringen.

9. Glaukom-Drainage nach einem der vorhergehenden Ansprüche, **dadurch gekennzeichnet, dass** das transversale Glied (10, 50, 110) eine Breite im Bereich von etwa 3 bis 4 mm hat.

10. Glaukom-Drainage nach einem der vorhergehenden Ansprüche, **dadurch gekennzeichnet, dass** die Drainage eine Öffnung (22, 72, 122) umfasst, um die Drainage an die tiefe Skleraklappe anzunähen.

11. Glaukom-Drainage nach einem der vorhergehenden Ansprüche, **dadurch gekennzeichnet, dass** die Gesamtlänge der Drainage im Bereich von etwa 2,5 bis 3,5 mm liegt.

12. Glaukom-Drainage nach einem der vorhergehenden Ansprüche, **dadurch gekennzeichnet, dass** die Vorderkante (14) im Wesentlichen geradlinig und so konfiguriert ist, dass sie auf ihrer gesamten Breite mit der Trabecula-Descemet-Membran in Kontakt ist.

13. Glaukom-Drainage nach einem der Ansprüche 1 bis 12, **dadurch gekennzeichnet, dass** die Vorderkante (54, 114) vorsteht, um die Trabecula-Descemet-Membran unter Spannung zu setzen.

14. Glaukom-Drainage nach einem der vorhergehenden Ansprüche, **dadurch gekennzeichnet, dass** die Vorderkante einen konvexen Teil (54, 114) aufweist.

15. Glaukom-Drainage nach Anspruch 13, **dadurch gekennzeichnet, dass** die vorstehende Vorderkante (54, 114) gebaucht ist und zwischen den Vorderkanten der Seitenarme angeordnet ist.

16. Glaukom-Drainage nach einem der vorhergehenden Ansprüche, **dadurch gekennzeichnet, dass** zwischen den Armen des Glieds (50) ein Loch (125) ausgebildet ist, das ein Ziel bildet, um die Mikropunktionen des Lasers zu positionieren.
